# EUROPEAN PATENT APPLICATION

(11) **EP 2 042 538 A1**
(43) Date of publication of application: **01.04.2009**
(21) Application number: 07116651.6
(22) Date of filing: 18.09.2007
(51) Int. Cl.: C08G 63/664, C08G 69/36, A61K 9/00

(54) **Amphiphilic copolymers and compositions containing such polymers**

(71) Applicant: Nirvana's Tree House, 6261 NJ Mheer (NL)
(72) Inventor: Heller, Jorge, Aswhland, 97520 (US); Pierre, Sebastien, 6226 GP, Maastricht (NL); Leeuw, de, Mike, 6261 NJ, Mheer (NL); Pieper, Jeroen, 6611 KT Overasselt (NL)
(74) Representative: DeltaPatents B.V.

(57) **Abstract**

Amphiphilic copolymer, containing at least a hydrophilic chain segment (A) and a hydrophobic chain segment (B), both segments being biodegradable. The copolymer may be an A(BA)ₓ, B(AB)ₓ or (AB)ₓ block copolymer, being an integer between 1 and 5. Copolymers with B main chains and grafted side chains made of A chain segments.
Compositions containing such polymers.

## Description

### Field of the invention.

The invention relates to amphiphilic copolymers, compositions containing the copolymers and at least one therapeutically active agent as well as implants containing the copolymers. The invention also relates to methods of treatment by administering the compositions to a human or animal body.

### Background of the invention.

Controlled release of therapeutically active agents is important in treatments of humans and animals.

In recent years, a number of polymers fabricated into devices as microspheres, microcapsules, liposomes, strands and the like have been developed for this reason. The active agent is incorporated into the interior of the devices and is after administration to the human or animal body slowly released by different mechanisms. U. S. Pat. Nos. 4,079,038, 4,093,709, 4,131,648, 4,138,344, 4,180,646, 4,304,767, 4,946,931 and 5,9689,543 disclose various types of polymers that may be used for the controlled delivery of active agents. The fabrication of such devices is in many cases cumbersome, expensive and may also suffer from irreproducibility in the release kinetics. Furthermore, in most cases an organic solvent is used which may have adverse effect on the therapeutic agent and there could also be residual solvent in the device, which in many cases is highly toxic. Moreover the administration of the solution or dispersion containing the devices is not patient friendly, due to the high viscosity of such solutions or dispersions. Further, such devices are not generally useful for the delivery of proteins that usually undergo a loss of activity during their incorporation into the solid polymer

An important improvement was found in the application of amphiphilic copolymers, containing at least one hydrophilic chain segment (A) and one hydrophobic chain segment (B). Such copolymers may form micelles or thermoreversible gels in water that may contain at least one therapeutically active agent.

Micelles of the amphiphilic copolymer have a number of useful attributes. For example when micelles having the correct size are used, which is usually below 40 nm, they will not extravasate in normal vasculature, but are able to extravasate in a tumor that normally has a leaky vasculature. Because of this it is possible to achieve a high concentration of antineoplastic agents in the tumor, without incurring excessive toxicity in normal tissues.

In addition to the usefulness as micelles in tumor targeting, micelles also find important applications in the solubilization of highly water insoluble drugs, since such drugs may be incorporated in the hydrophobic core of the micelle.

The use of micelles in tumor targeting and solubilization of highly water-insoluble drugs has been extensively described by V. P. Torchilin, Structure and design of polymeric surfactant-based drug delivery systems", J. Controlled Release 73 (2001) 137-172, and by V. P. Torchilin, "Polymeric Immunomicelles: Carriers of choice for targeted delivery of water-insoluble pharmaceuticals", Drug Delivery Technology, 4 (20004) 30-39.

It is also possible for amphiphilic copolymers having a certain composition to form a so-called thermogel. Such a copolymer has the unique property that at room temperature it is water-soluble and at the body temperature of 37°C it is water-insoluble and forms a gel.

The composition containing the copolymer and the therapeutically active agent may be administered at room temperature as a low viscosity solution in water, using a small gauge needle, thus minimizing discomfort for the patient. Once at body temperature the composition will form a well-defined gel that will be localized at the desired site within the body. Further, such materials are also uniquely suited for use with a protein as the therapeutically active agent since the protein is simply dissolved in the same solution that contains the amphiphilic copolymer and the solution is injected, without affecting the properties of the protein.

The therapeutically active agent is slowly released by diffusion, or by a combination of diffusion and erosion, from the micelles or the thermogels made of amphiphilic copolymers. Ultimately, the amphiphilic copolymers has to fall apart into small fragments that can be metabolized or removed from the body.

Micelles based on poly(ethylene glycol) and poly(*D,L*-lactic acid) have been investigated by J. Lee, "Incorporation and release behaviour of hydrophobic drug in functionalized poly(D,L-lactide)-block poly(ethylene oxide) micelles" J. Controlled Release, 94 (2004) 323-335. Micelles based on poly(ethylene glycol) and poly(β-benzyl-*L*-aspartate) have been investigated by Kataoka, G. Kwon, "Block copolymer micelles for drug delivery: loading and release of doxorubicin" J. Controlled Release, 48 (1997) 195-201. Micelles based on poly(ethylene glycol) and poly(ortho ester) have been described by Toncheva et. al., "Use of block copolymers of poly(ortho esters) and poly(ethylene glycol) micellar carriers as potential tumour targeting systems", J. Drug Targeting, 11 (2003) 345-353.

Thermogels have been extensively investigated. The most extensively investigated thermogelling polymer is poly(*N*-isopropyl acrylamide). This polymer is soluble in water below 32°C and sharply precipitates as the temperature is raised above 32°C. This temperature is known as the lower critical solution temperature, or LCST. Thus, such a polymer could be injected at room temperature as a low viscosity solution using a small bore needle, and once in the tissues, it would precipitate, forming a well-defined depot. However, such polymers are non-degradable. Such polymers were extensively described by Hoffman, in L. C. Dong et. al., "Thermally reversible hydrogels: III. Immobilization of enzymes for feedback reaction control", J. Controlled Release, 4 (1986) 223-227.

Thermogels using poly(lactide-*co*-glycolide) copolymers as the hydrophobic segment and poly(ethylene glycol) as the hydrophilic segment have been extensively investigated and are described in a number of patents and publications: U.S. Pat. Nos. 5,702,717, 6,004,573, 6,117,949, 6,201,072 B1. G. Zentner, J. Controlled Release, 72 (2001) 203-215.

Thermogels based on amphiphilic graft copolymers having a hydrophobic poly(lactide-*co*-glycolide) backbone and poly(ethylene glycol) grafts, or a poly(ethylene glycol) backbone and poly(lactide-*co*-glycolide) grafts have also been described. Thermogelling polymers with hydrophilic backbones: PEG-g-PLGA, Macromolecules, 33 (2000) 8317-8322.

### Summary of the invention.

A problem with known amphiphilic copolymers is that the copolymers are not fully degraded and removed from the body, and high molecular weight degradation products, for example poly(ethylene glycol) (PEG) remain in the body and can accumulate inside cells.

The accumulation problem can be solved by constructing an amphiphilic copolymer, containing at least one hydrophilic chain segment (A) and one hydrophobic chain segment (B), both the segment (A) and the segment (B) being biodegradable.

### Detailed description of the invention.

The amphiphilic copolymer may be a block copolymer or a graft copolymer. In the case of a block copolymer, the copolymer may be an A(BA)ₓ, B(AB)ₓ or (AB)ₓ block copolymer, block A being the hydrophilic chain segment (A), block B being the hydrophobic chain segment (B) and x being an integer between 1 and 5. It is also possible that the amphiphilic copolymer is a graft copolymer where the polymeric backbone of the copolymer is the hydrophilic chain segment (A) or the hydrophobic chain segment (B), and where the polymeric grafted chains are the hydrophobic chain segments (B) when the backbone is hydrophilic, and the hydrophilic segments (A) when the backbone is hydrophobic.

Biodegradation in the context of the present invention refers to the degradation, disassembly or digestion of the amphiphilic copolymers by action of the biological environment, including action of living organisms and most notably at physiological pH and temperature. A principal mechanism for biodegradation in the present invention is the hydrolysis of linkages between and within the monomer units of the amphiphilic copolymers. Specific reactions include but are not limited to ester hydrolysis (chemical or enzymatic) and proteolysis of the amide bonds of amino-acids chains and reactions yielding natural-occurring molecules including but not limited to lactic acid, glycolic acid and amino-acids.

In a preferred embodiment the hydrophilic chain segments (A) contain glutamine or aspartamine groups.

More preferably the hydrophilic chain segments (A) contain monomeric units of *N-*(2-hydroxyalkyl)-*L*-glutamine, *L*-glutamic acid, *N*-(2-hydroxyalkyl)-*L*-aspartamine, *L-*aspartic acid or glycolic acid or combinations thereof. Even more preferably the segment (A) contains monomeric units of *N*-(2-hydroxyethyl)-*L*-glutamine (such a segment also being referred to as poly[*N*-(2-hydroxyethyl)-*L*-glutamine]). Still more preferably the hydrophilic chain segment is poly[*N*-(2-hydroxyalkyl)-*L*-glutamine], poly[*L*-glutamic acid], poly[*N*-(2-hydroxyalkyl)-*L*-aspartamine], poly[*L*-aspartic acid] or polyglycolide.

Chain segments containing the monomeric units described above are biodegradable, hydrophilic and they can be prepared in a range of well-defined molecular weights. This enables the fabrication of copolymers that have a well-defined structure, so that well-defined micelles or thermogels can be formed from the copolymers and moreover good reproducibility in the release kinetics of the therapeutically active agent may be achieved.

The hydrophobic chain segments (B) preferably contain acetal groups, ortho ester groups or combinations thereof. Examples of monomers used to make hydrophobic segments containing ortho ester groups include but are not limited to those in formula IX as defined below.

Other hydrophobic segments for example poly-*L*-lactide, poly-*D,L*-lactide, poly(lactide-*co*-glycolide), polyanhydride, polyphosphazene, polyketals and the like may also be used. Combinations of any of those hydrophobic segments may also be used.

Preferred amphiphilic copolymers according to the invention include poly[N-(2-hydroxyalkyl)-L-glutamine]-polyacetal, poly[N-(2-hydroxyalkyl)-*L*-glutamine]-polyacetal-poly[N-(2-hydroxyalkyl)-*L*-glutamine], polyacetal-poly[N-(2-hydroxyalkyl)-*L*-glutamine]-polyacetal block copolymers, polyacetal-poly[N-(2-hydroxyalkyl)-*L*-glutamine] graft copolymers, poly[N-(2-hydroxyalkyl)-*L*-glutamine]-poly(ortho ester), poly[N-(2-hydroxyalkyl)-*L*-glutamine]-poly(ortho ester)-poly[N-(2-hydroxyalkyl)-*L*-glutamine], poly(ortho ester)-poly[N-(2-hydroxyalkyl)-*L*-glutamine]-poly(ortho ester) block copolymers, poly(ortho ester)-poly[N-(2-hydroxyalkyl)-*L*-glutamine] graft copolymers, poly[N-(2-hydroxyalkyl)-*L*-aspartamine]-polyacetal, poly[N-(2-hydroxyalkyl)-*L-*aspartamine]-polyacetal-poly[N-(2-hydroxyalkyl)-*L*-aspartamine], polyacetal-poly[N-(2-hydroxyalkyl)-*L*-aspartamine]-polyacetal block copolymers, polyacetal-poly[N-(2-hydroxyalkyl)-*L*-aspartamine] graft copolymers, poly[N-(2-hydroxyalkyl)-*L*-aspartamine]-poly(ortho ester), poly[N-(2-hydroxyalkyl)-*L*-aspartamine]-poly(ortho ester)-poly[N-(2-hydroxyalkyl)-*L*-aspartamine], poly(ortho ester)-poly[N-(2-hydroxyalkyl)-*L*-aspartamine]-poly(ortho ester) block copolymers, poly(ortho ester)-poly[N-(2-hydroxyalkyl)-*L-*aspartamine] graft copolymers, poly(L-glutamic acid)-polyacetal, poly(L-glutamic acid)-polyacetal-poly(L-glutamic acid), polyacetal-poly(L-glutamic acid)-polyacetal block copolymers, polyacetal-poly(L-glutamic acid) graft copolymers, poly(L-glutamic acid)-poly(ortho ester), poly(L-glutamic acid)-poly(ortho ester)-poly(L-glutamic acid), poly(ortho ester)-poly(L-glutamic acid)-poly(ortho ester) block copolymers, poly(ortho ester)-poly(L-glutamic acid) graft copolymers, poly(L-aspartic acid)-polyacetal, poly(L-aspartic acid)-polyacetal-poly(L-aspartic acid), polyacetal-poly(L-aspartic acid)-polyacetal block copolymers, polyacetal-poly(L-aspartic acid) graft copolymers, poly(L-aspartic acid)-poly(ortho ester), poly(L-aspartic acid)-poly(ortho ester)-poly(L-aspartic acid), poly(ortho ester)-poly(L-aspartic acid)-poly(ortho ester) block copolymers, poly(ortho ester)-poly(L-aspartic acid) graft copolymers, polyglycolide-polyacetal, polyglycolide-polyacetal-polyglycolide, polyacetal-polyglycolide-polyacetal block copolymers, polyacetal-polyglycolide graft copolymers, polyglycolide-poly(ortho ester), polyglycolide-poly(ortho ester)-polyglycolide, poly(ortho ester)-polyglycolide-poly(ortho ester) block copolymers, poly(ortho ester)-polyglycolide graft copolymers, and combinations thereof.

### Examples of suitable polymers according to the invention are also polymers shown in the formulas I to VIII.

### Formula I. Graft copolymer.

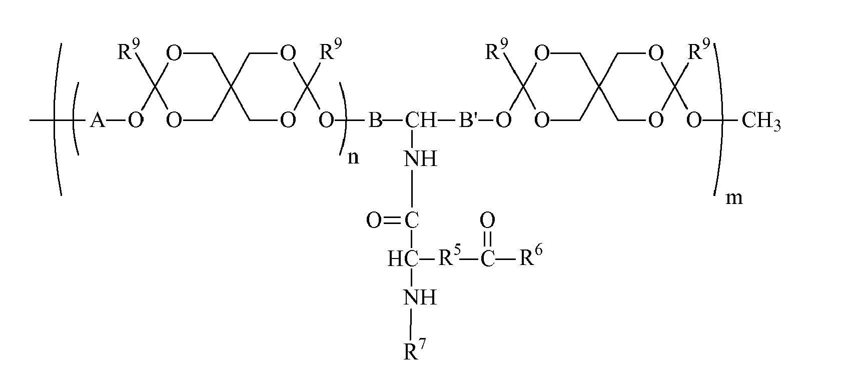

where:
R' is a C₁ to C₄ alkyl chain.
n and x are independently integers between 1 and 50.
m is an integer between 2 and 500.
B and B' are C₁ to C₅ alkyl chains.
A and A' can be R², R³, or a mixture thereof.
R² is selected from: 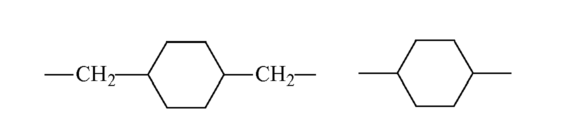 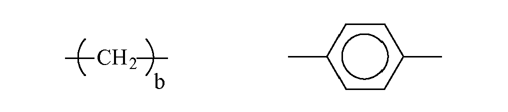
where b is an integer between 1 and 12.

### R³ is:

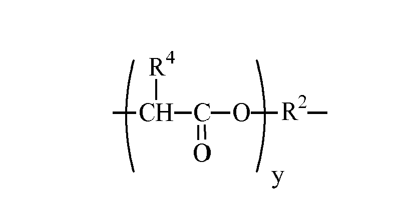

where R⁴ is H or a C₁ to C₆ alkyl chain and y is an integer between 1 and 10.
R⁵ = (CH₂)_{z}
z is integer between 1 and 6.
R⁶ = OH, OCH₃ , NH-(CH₂-)_{z}OH, NH-(CH₂-CH₂-O-)_{z}H, NH-CH₂-CH(OH)-CH₂-OH,
NH-(CH₂-)_{z}O-CO-CH=CH₂, NH-(CH₂-)_{z}O-CO-C(CH₃)=CH₂, N-(CH₃)₂ and mixtures of those...
z is an integer between 1 and 6.
R⁷ = CO-CH₃ , CO-CH=CH₂, CO-C(CH₃)=CH₂ and mixtures of those...

### Formula II. An AB block copolymer.

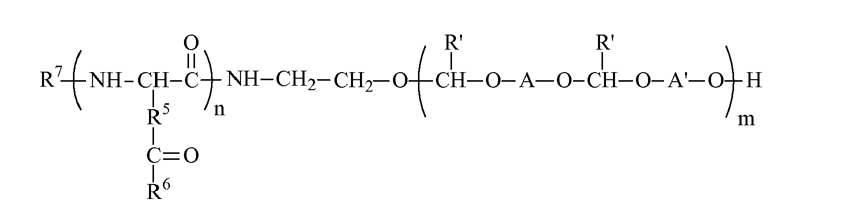

where n, m, A, A', R⁵, R⁶ and R⁷ are defined as stated in formula I.

### Formula III. An ABA block copolymer.

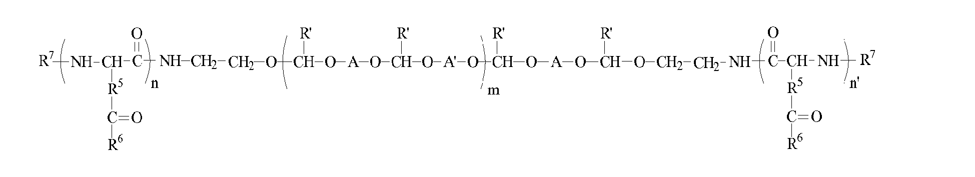

where n, m, A, A', R⁵, R⁶ and R⁷ are defined as stated in formula I.
n' is an integer between 1 and 50.

### Formula IV. A BAB block copolymer.

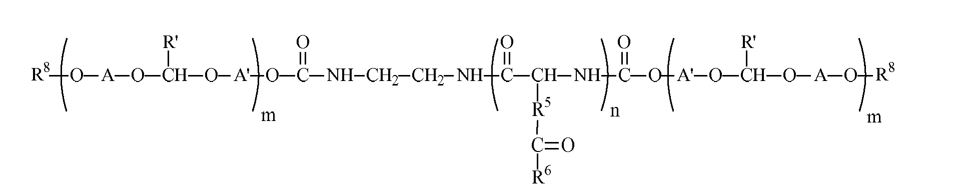

where n, m, A, A', R⁵ and R⁶ are defined as stated in formula I.
R⁸ = CH=R' , CH(R')-O-CH₃ ...

### Formula V. A graft copolymer.

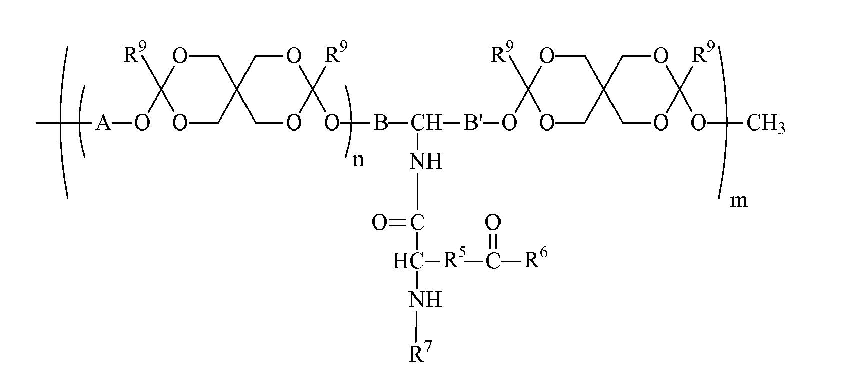

where n, m, x, A, B, B', R⁵, R⁶ and R⁷ are defined as stated in formula I.
R⁹ = -H, -CH₃, -(CH₂)_{c}-CH₃.
c is an integer between 1 and 3.

### Formula VI. An AB block copolymer.

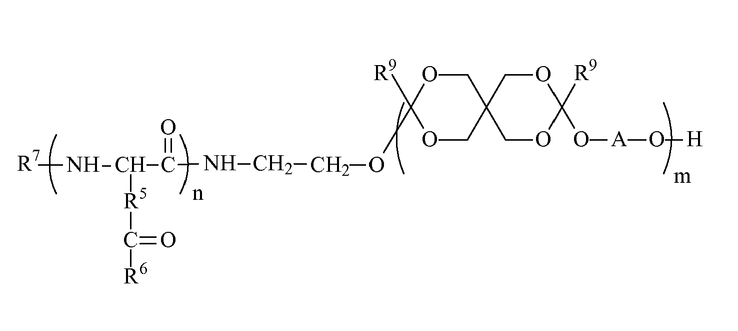

where n, m, A, R⁵, R⁶ and R⁷ are defined as stated in formula I.
R⁹ is defined as stated in formula V.

### Formula VII. An ABA block copolymer.

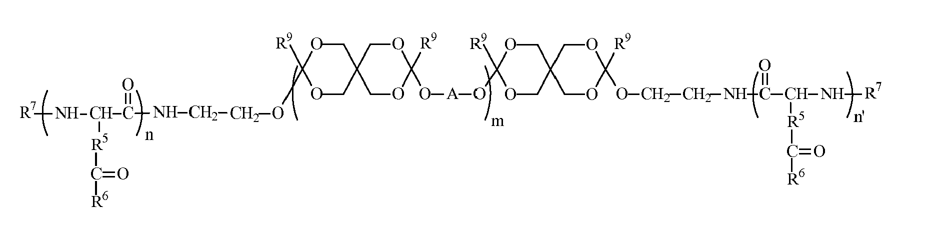

where n, m, A, R⁵, R⁶ and R⁷ are defined as stated in formula I.
n' is an integer between 1 and 50.
R⁹ is defined as stated in formula V.

### Formula VIII. A BAB block copolymer.

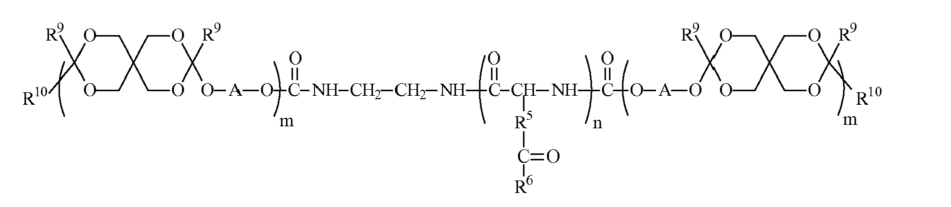

where n, m, A, R⁵ and R⁶ are defined as stated in formula I.
R⁹ is defined as stated in formula V.
R¹⁰ = O-CH₃ ...

### Formulas IX. Ketene acetal monomeric units for poly(ortho ester) hydrophobic segments (respectively structures 1, 2 and 3).

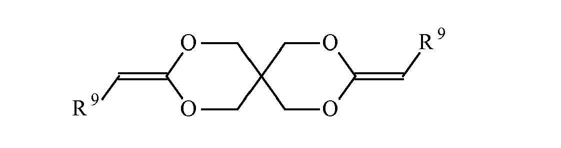

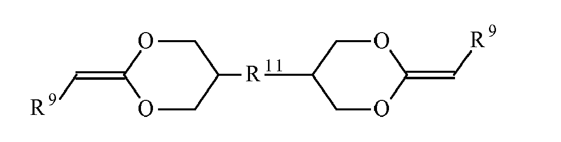

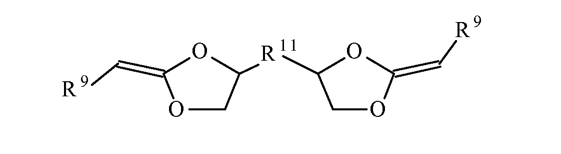

R⁹ is defined as stated in formula V.
R¹¹ = -(CH₂)_{d}- , -(CH₂)ₑ-O-(CH₂)_{f}-
d is an integer between 1 and 10, e and f are independent integer between 1 and 6.

The hydrophilic chain segment (A) containing the [*N*-(2-hydroxyethyl)-*L-*glutamine] may be prepared by polymerization of suitably substituted *N*-carboxy anhydrides using amino groups on the polymer chains to initiate the polymerization.

Hydrophobic chain segments (B) that contain acetal groups may be prepared by a transacetalization reaction that is an equilibrium reaction and must be driven to high molecular weight by removing the low molecular weight by-products, usually alcohols. Preferably these chain segments are prepared by the reaction of a polyol and a divinyl ether as described by J. Heller et. al., "Preparation of polyacetals by the reaction of divinyl ethers and polyols" J. Polymer Sci., Polymer Letters Ed., 18 (1980) 293-297 and in U.S. Patent No. 4,713,441.

Chain segments containing the ortho ester groups may be prepared by the addition of polyols to the diketene acetal 3,9-diethylidene-2,4,8,10-tetraoxaspiro[5.5] undecane (DETOSU, see Structure 1 on Figure 9). Their preparation and applications have been extensively reviewed by Heller, Poly (Ortho Esters), Advances in Polymer Science, Vol.107, (1993) 41-92 and Heller, et. al., Poly(ortho esters): Synthesis, characterization, properties and uses, Adv. Drug Delivery Rev., 54 (2002) 1015-1039.

Chain segments containing the ortho ester group can also be prepared using Structures 2 and 3 of Formula IX. Structure 2 can be prepared as described by Newsome et. al., US 6,863,782 B2. Structure 3 can be prepared as described by Crivello et. al., Ketene acetal monomers: synthesis and characterization, J. Polymer Sci., Part A: Polymer Chem., 34 (1996) 3091-3102.

The chain segment containing *N*-(2-hydroxyethyl)-*L*-glutamine may be incorporated into the amphiphilic block copolymers by two distinctly different procedures. In one procedure, a hydrophobic, amine-terminated block is prepared, preferably a polyacetal, a poly(ortho ester), or a combination of the two groups with the two terminal amino groups being used to initiate the polymerization of a suitably substituted *N*-carboxyanhydride. Graft copolymers with a hydrophobic backbone may be prepared using a polyacetal, a poly(ortho ester) or a combination of the two groups with pendant amino groups and the polymerization of a suitably substituted *N-*carboxyanhydride initiated by the pendant amino groups.

In a second procedure, block or graft copolymers are formed by coupling a suitably monosubstituted poly[*N*-(2-hydroxyethyl)-*L*-glutamine] to a polymer, preferably a polyacetal, a poly(ortho ester), or a combination of the two groups containing amino end-groups, or pendant amino groups. Of these two procedures, the initiation of a suitably substituted *N*-carboxyanhydride by terminal, or pendant amino groups of a polyacetal, a poly(ortho ester) or a combination of the two groups, is preferred since the difficult removal of unreacted poly[*N*-(2-hydroxyethyl)-*L*-glutamine] segments is not required.

The copolymers of this invention will find utility in any of the uses for which biodegradable polymers are useful, including such uses as vehicles for the sustained release of therapeutically active agents, orthopedic implants, degradable sutures, and the like, they will also find particular utility in applications where their nature as block and graft copolymers having both hydrophilic and hydrophobic segments confers a special benefit, and those uses will be addressed in greater detail below.

For some applications special moieties may have to be introduced into the polymer chains that allow chemical reactions to occur between the polymer chains to achieve polymer crosslinking. Crosslinking is usually carried out in order to modify the mechanical properties and degradation profile of polymers. There are a number of ways known to those skilled in the art to introduce these moieties into the polymers described above via terminal hydroxy- and amino- groups, known as functionalisation of the main chain and/or sidechains. These moieties may include, but are not limited, to acrylates, methacrylates, vinyl groups, styryl groups, acrylamides, methacrylamides, thiols, thiol-ene dual systems. The activation and intermolecular reaction of these moieties is usually caused by a radiation source, an external chemical reaction or stimulus, or a combination thereof. Radiation examples include, but are not limited, to heat, infrared sources, ultra-violet sources, electron-beam sources, micro-waves sources, x-ray sources, visible light sources [monochromatic or not] and gamma-rays. External reaction, or stimulus include, but are not limited, to pH, oxidation/reduction reactions, reactions with a chemical agent present in vivo (gas, protein, enzymes, antibody etc), reaction with a chemical added to the composition upon introduction into the body, known as dual systems, for example a molecule containing two or more reactive groups.

The invention also relates to compositions containing at least one amphiphilic copolymer of the present invention and at least one therapeutically active agent.

By therapeutically active agents people skilled in the art refer to any set of molecules, cells or cell materials able to prevent, slow down or cure a disease. Therapeutically active agents include but are not limited to proteins, enzymes, peptides, nucleic acid sequences such as DNA and RNA, antigens, antibodies, viruses, virus-based materials, cells, cell substructures, synthetic drugs, natural drugs and substances derived from these.

### Micellar Systems.

In one preferred embodiment the composition contains micelles of the copolymer with the therapeutically active agent(s) being entrapped in the micelles.

When the copolymers are placed in water, in which the hydrophilic segment is soluble and the hydrophobic segment is insoluble, the polymer chains may spontaneously self-aggregate to form micellar structures depending on their concentration.

One major utility of such micellar structures resides in their ability to entrap and solubilize hydrophobic drugs in the hydrophobic core. Such entrapment can be carried out in a number of ways. The drug may be added to the aqueous media containing the micelles and incorporated by simple stirring, by heating to moderate temperatures or by ultrasonification. Alternately, a drug dissolved in a volatile organic solvent is added to a water solution of preformed micelles with a subsequent solvent evaporation from the system.

Efficient entrapment of hydrophobic drugs requires a highly hydrophobic core. The high hydrophobicity of polyacetals, poly(ortho esters) or their copolymers allows the preparation of micellar systems with significantly enhanced entrapment efficiency relative to other biodegradable segments such as poly(lactic-*co*-glycolic) acids copolymers.

While any of the anticancer agents that can be incorporated in micellar structures are suitable for this use, anticancer agents that are particularly suitable for micellar tumor targeting are those with low water solubility such as doxorubicin, daunorubicin, epirubicin, mitomicin C, paclitaxel, cis-platin, carboplatin, and the like. Other agents may include anticancer proteins such as neocarzinostatin, *L-*aspariginase, and the like and photosensitizers used in photodynamic therapy. In addition to the usefulness as micelles in tumor targeting, micelles also find important applications in the solubilization of highly water insoluble drugs, since such drugs may be incorporated in the hydrophobic core of the micelle.

### Thermogels.

In another preferred embodiment the composition contains the copolymer according to the invention and the therapeutically active agent as a solution in water, the solution having a lower critical solution temperature (LCST) below 37°C.

Such polymers are water-soluble below their LCST, also known as gel temperature, due to strong hydrogen bonding between the hydrophilic part of the chains and water, but above the LCST value hydrogen interactions are weakened and hydrophobic interactions between the hydrophobic domains of the polymer become dominant with consequent precipitation of the polymer resulting in gelation.

The LCST value depends on the balance of hydrophilic and hydrophobic portions of the block, or graft copolymer and can be adjusted by varying this balance. It also depends on the concentration of the block, or graft copolymer in water. Materials having particular usefulness for therapeutic applications are those where the LCST value is between 22 and 37°C since such materials will be soluble in water at room temperature and form a gel at the body temperature of 37°C.

One of the desirable features of thermogels is the ability to administer thermogel formulations using a small bore needle resulting in significantly less pain on administration relative to the administration of microspheres, microcapsules, strands, or other solid drug-releasing devices. This is due to the water solubility of thermogels at room temperature, and the relatively low viscosity of the aqueous solution making the use of small-bore needles possible.

Another important and unique feature is the ability to deliver therapeutically active agents at a controlled rate and without loss of biological activity. In this application, the polymer according to the invention is dissolved in an appropriate volume of water and the peptide, protein or nucleic acid sequence is dissolved in the same solution. The mixture is then injected in the desired body site, where it gels, entrapping the peptide, protein or nucleic acid sequence in the gelled material. It will be appreciated that these are extremely mild conditions since active agents are only exposed to water and temperatures no higher than the body temperature of 37°C.

This method is greatly superior to conventional methods of protein incorporation into solid polymers that require harsh conditions such as elevated temperatures, and/or organic solvents, or mixtures of organic solvents ad water that usually results in loss of protein activity.

This method is particularly useful for the delivery and dosing of therapeutically active agents in applications including but not limited to injections of the thermogels containing the proteins mentioned above into articulate cartilage, pericardium, cardiac muscles, sclera and the vitreous body of the eye.

The LCST behaviour also gives advantages when building composite devices. They can be built by using several thermogels with different LCST (always below 37°C). Upon implantation the in vitro degradation and release of actives can be tuned depending on their LCST and chemical structures.

In a further preferred embodiment the therapeutically active agent is a growth factor. Such a composition is very suitable for use in the treatment of intervertebral discs. This is because the composition will gel and hold the active agent in place over a period in time, releasing it in a slower manner than straight injection of a non-gelling solution. Further the gel-forming polymers will be completely broken down after having completed their function. This is especially important in the area of intervertebral discs, where there is less metabolistic action.

Preferably as growth factor at least one compound is used of the group consisting of transforming growth factor beta-3, osteogenic protein 1, bone morphogenic protein 2 and 7. Although less preferred it is also possible to use compositions containing thermogels in general and a transforming growth factor. Such a composition at least has the advantage of the slow release of the growth factor. *Bioerodible Copolymer. Matrix for Controlled Delivery and Tissue Engineering*

The invention also relates to an implant containing the polymer according to the invention. In certain uses it is desirable to have a material that has improved mechanical properties relative to thermogelling materials. To this effect, solid polymers can be prepared that are useful in a number of applications, for example orthopedic applications such as fracture fixation, or repair of osteochondral defects and the like. The solid polymer can be readily fabricated into a number of shapes and forms for implantation, insertion or placement on the body or into body cavities or passageways. For example, the block, or graft copolymer may be injection molded, extruded or compression molded into a thin film, or made into devices of various geometric shapes or forms such as flat, square, round, cylindrical, tubular, discs, rings and the like. Rod, or pellet-shaped devices may be implanted using a trocar, and these, or other shapes, may be implanted by minor surgical procedures. Alternatively, a device may be implanted following a major surgical procedure such as tumor removal in the surgical treatment of cancer. The implantation of polymer wafers containing anticancer agents is described for example, in Brem et. al., US Pat. Nos. 5,626,862 and 5,651,986 and references cited therein; and the block and graft copolymers will find utility in such applications.

### Tissue engineering

Tissue engineering applications using thermogels made with copolymers described in this invention include but are not limited to nerve growth or repair, cartilage growth or repair, bone growth or repair, muscle growth or repair, skin growth or repair, secreting gland repair, ophtalmic repair. It should be underlined that thermogels may be used as such or as a part of a bigger implant, scaffold or structure.

Thermogels with LCST below 37°C may also be used as temporary void fillers in case of significant trauma, to prevent adhesion of damage tissues and scar tissue formation while waiting for corrective and reconstructive surgery. Void filling could be performed easily by injecting the thermogels and removal could be performed via cutting, scraping or suction after cooling down the area to liquefy the thermogel. Other benefits of using voidfillers may include but are not limited to: preventing contamination from outside, preventing infection, preventing surrounding tissue necrosis or alteration, inducing specific tissue formation (bone, cartilage, muscle, nerve, skin etc.), helping to maintain structural integrity of the surrounding tissues by itself or by combination with other known scaffolds or structures, trapping specific natural or foreign molecules.

Thermogelling polymers of this invention also exhibit little or no swelling upon gelation when reaching the LCST. This is useful for some implant or tissue-engineering applications such as intra-ocular and intra-cranial surgery, where swelling after implantation can increase pressure for example on nerves, organs or bones and thus can cause damage. The difficulty to find proper materials for orbital reconstruction is described by G. Enislidis "Treatment of orbital fractures: the case for treatment with bioresorbable materials" J. Oral Maxillofacial Surgery, 62 (2004) 869-872.

The invention is further explained in the experimental part, without being limited to the examples.

### Example 1: synthesis of an amino-terminated polyacetal.

The reaction was carried out in a glove box. 2.5 g (0.013 mol) 1,4-cyclohexanedimethanol divinyl ether, 1.6 g (0.011 mol) trans-1,4-cyclohexanedimethanol, and 0.23 g (0.82 mmol) of Fmoc-aminoethanol were dissolved in 10 ml of dry tetrahydrofuran. 0.2 ml of the catalyst, p-toluenesulfonic acid (10 mg/ml in tetrahydrofuran) was added under stirring and the reaction was carried out at room temperature for 5 h. Then 2.0 ml piperidine was added and the solution was stirred for another 2 h at room temperature. The product was purified by dialysis in tetrahydrofuran (molecular weight cut-off: 1000 Da) for 3 days and isolated by evaporation of the solvent. The prepolymer was characterized by ¹H NMR (CDCl₃) and GPC chromatography (in tetrahydrofuran, with polystyrene standards).

### Example 2: synthesis of an amino-terminated poly(ortho ester).

The reaction was carried out in a glove box. 2.0 g (0.0094 mol) DETOSU, 1.24 g (0.0085 mol) trans-1,4-cyclohexanedimethanol, and 0.18 g Fmoc-aminoethanol were dissolved in 20 ml tetrahydrofuran. Two drops of the catalyst, p-toluenesulfonic acid (10 mg/ml in tetrahydrofuran) were added under stirring and the reaction was carried out at room temperature for 2 h. Then 4.0 ml piperidine was added and the solution is stirred for another 2 h at room temperature. The product was purified by dialysis in tetrahydrofuran (molecular weight cut-off: 1000 Da) for 3 days and isolated by evaporation of the solvent. The product was characterised by ¹H NMR (CDCl₃) and GPC chromatography (in tetrahydrofuran, with polystyrene standards).

### Example 3: synthesis of an amino-terminated poly[N-(2-hydroxyethyl)-L-glutamine].

2.0 g N-carboxyanhydride of γ-trichloroethyl-L-glutamate (TCEG-NCA) were dissolved in 20 ml dry 1,2-dichloroethane and the resulting solution was cooled down to 10°C. 0.099 g 1-triphenylmethylaminoethylamine (i.e. 5 mole % with respect to TCEG-NCA) was dissolved in 2 ml 1,2-dichloroethane and added to the solution of TCEG-NCA. Polymerization of TCEG-NCA proceeded by maintaining the temperature at 10° C and was complete after 2h (determined by infrared spectroscopy). Then a three-fold molar excess of acetic anhydride and equimolar quantity of triethylamine was added and the reaction mixture was stirred for 2 h at room temperature. The solution was precipitated in pentane and the polymer produced was isolated by filtration and dried under vacuum. Its molecular weight was determined by 1 H NMR (DMF-d7) and gel permeation chromatography (in tetrahydrofuran, with polystyrene standard). 1.0 g (3.8 mmol) of the polymer obtained above was dissolved in 10 ml dry N,N-dimethylformamide. This solution was cooled down to 10°C and 0,69 ml (11.5 mmol) ethanolamine and 0.36 g (3.8 mmol) 2-hydroxypyridine were then added. The reaction was followed by infrared spectroscopy and was complete after 2 h. The resulting aminolysed polymer was isolated by precipitation in ether, filtered, dried under vacuum and then purified by gel filtration on Sephadex G-25 (water as eluent) and isolated by lyophilization. The purified polymer was characterised by ¹H NMR (D₂O) and gel permeation chromatography (in water, with dextran standards).
1.0 g of the aminolysed polymer was dissolved in 10 ml trifluoroacetic acid and stirred at room temperature for 30 min. Trifluoroacetic acid was then removed by evaporation under vacuum. The resulting polymer was dissolved in water and centrifugated, then the supernatant was purified by GPC (Sephadex G-25, water as eluent) and isolated by lyophilization.

### Example 4: synthesis of a poly[N-(2-hydroxyethyl)-L-glutamine]-polyacetal diblock copolymer (PHEG-PA).

2.0 g 1,4-butanediol divinyl ether and 0.11 g Fmoc-aminoethanol were dissolved in 6 ml of dry tetrahydrofuran. 0.1 ml of the catalyst, p-toluenesulfonic acid (10 mg/ml in tetrahydrofuran) was added under stirring and the reaction was carried out at room temperature for 5 h. Then 1.2 ml piperidine was added and the solution was stirred for 2 h at room temperature. The product was purified by dialysis in tetrahydrofuran (molecular weight cut-off: 1000 Da) for 3 days and isolated by evaporation of the solvent. The product was characterised by ¹H NMR (CDCl₃) and GPC chromatography (in tetrahydrofuran, with polystyrene standards). 2.6 g N-carboxyanhydride of trichloroethyl-*L*-glutamate were dissolved in 10 ml dry chloroform. 1.5 g prepolymer was dissolved in 5 ml dry chloroform and added to the solution. The reaction was followed by infrared spectroscopy and was complete after 3 h mixing. 0.4 ml acetic anhydride and 0.6 ml triethylamine were added and the stirring continued for 2 h. At the end the product was precipitated in pentane, filtered, and dried under vacuum. 3.5 g copolymer were dissolved in dry N,N-dimethylformamide and cooled to 10°C. 1.7 ml 2-aminoethanol and 0.8 g 2-hydroxypyridine were added and the solution was stirred for 2 h. The reaction was followed by infrared spectroscopy. At the end the solvent was evaporated, the product was dissolved in water and purified by preparative GPC (Sephadex G-25, water as eluent). The final copolymer was isolated by lyophilization.

### Example 5: synthesis of a poly[N-(2-hydroxyethyl)-L-glutamine]-poly(ortho ester)-poly[N-(2-hydroxyethyl)-L-glutamine] triblock copolymer (PHEG-POE-PHEG).

3.0 g of the prepolymer from example 2 were dissolved in 10 ml dry chloroform. 5.3 g N-carboxyanhydride of trichloroethyl-L-glutamate were dissolved in 50 ml dry chloroform and added to the solution. The mixture was stirred for 2 h. At the end of the reaction which was followed by infrared spectroscopy, 1.7 ml acetic anhydride and 2.2 ml triethylamine were added and the stirring continued for 2 h. At the end, the product was precipitated in hexane, filtered, and dried under vacuum.
7.0 g of the copolymer were dissolved in 70 ml dry N,N-dimethylformamide and cooled to 10°C. 3.6 ml 2-aminoethanol and 1.7 g 2-hydroxypyridine were added and the solution was stirred for 2 h. The reaction was followed by infrared spectroscopy. At the end the solvent was evaporated, the product was dissolved in water and purified by preparative GPC (Sephadex G-25, water as eluent). The final copolymer was isolated by lyophilization.

### Example 6: synthesis of a polyacetal-poly[N-(2-hydroxyethyl)-L-glutamine]-polyacetal triblock copolymer (PA-PHEG-PA).

2.5 g (0.013 mol) 1,4-cyclohexanedimethanol divinyl ether and 1.9 g (0.013 mol) trans-1,4-cyclohexanedimethanol were dissolved in 10 ml of dry tetrahydrofuran. 0.2 ml of the catalyst, p-toluenesulfonic acid (10 mg/ml in tetrahydrofuran) was added under stirring and the reaction was carried out at room temperature for 5 h. Then 2 ml piperidine was added and the solution was stirred for another 2 h at room temperature. The product was purified by dialysis in tetrahydrofuran (MWCO 1000) for 3 days and isolated by evaporation of the solvent. The PA prepolymer was characterised by 1 H NMR (CDCl₃) and GPC chromatography (in tetrahydrofuran, with polystyrene standards).
4.4 g (0.013 mol) PA prepolymer and 13.3 g (0.052 mol) N,N'-disuccinimidyl carbonate were dissolved in 60 mL dry tetrahydrofuran. 9.1 mL (0.052 mol) diisopropylethylamine were mixed with 10 mL dry tetrahydrofuran and added slowly to the prepolymer solution at room temperature. After 8 h, the succinimidyl-modified PA prepolymer was purified by dialysis in tetrahydrofuran (MWCO 1000) for 3 days and isolated by evaporation of the solvent. The modified PA prepolymer was characterised by 1 H NMR (CDCl₃) and GPC chromatography (in tetrahydrofuran, with polystyrene standards). 5.2 g (0.013 mol) modified PA prepolymer and 2.6 g (6.5 mmol) amino-terminated poly[*N*-(2-hydroxyethyl)-*L*-glutamine] were dissolved in 100 mL of 10 mM phosphate buffered saline (PBS) at pH 7.4. After 24 h of stirring at room temperature, the final PA-PHEG-PA copolymer was dialyzed to remove the PBS buffer (molecular weight cut-off: 1000 Da) and purified by subsequent preparative GPC (Sephadex G-25, water as eluent). The final PA-PHEG-PA copolymer was recovered by lyophilization.

### Example 7: synthesis of a poly(ortho ester) with pendant [N-(2-hydroxyethyl)-L-glutamine] groups (POE~PHEGs).

1.0 g (0,0047 mol) 3,9-diethylidene-2,4,8,10-tetraoxospiro[5.5]undecane (DETOSU), 0,47 g (3.3 mmol) trans-1,4-cyclohexanedimethanol and 0,44 g (1.4 mmol) Fmocserinol were dissolved in 6 ml tetrahydrofuran. One drop of the catalyst, p-toluenesulfonic acid (10 mg/ml in tetrahydrofuran) was added under stirring and the reaction was carried out at room temperature for 2 h. Then 1.2 ml piperidine was added and the solution was stirred for 2 h at room temperature. The product was purified by dialysis in tetrahydrofuran (molecular weight cut-off: 1000 Da) for 3 days and isolated by evaporation of the solvent. The product was characterized by ¹H NMR (CDCl₃) and GPC chromatography (in tetrahydrofuran, with polystyrene standards).
1.5 g of the prepolymer was dissolved in 10 ml dry chloroform. 4.7 g N-carboxyanhydride of trichloroethyl-*L*-glutamate were dissolved in 50 ml dry chloroform and added to the solution. The mixture was stirred for 3 h. At the end of the reaction which is followed by infrared spectroscopy, 2.4 ml acetic anhydride and 3.0 ml triethylamine were added and the stirring continued for 2 h. At the end, the product was precipitated in hexane, filtered, and dried under vacuum.
4.0 g of the copolymer were dissolved in dry N,N-dimethylformamide and cooled to 10°C. 2.0 ml 2-aminoethanol and 0.9 g 2-hydroxypyridine were added and the solution was stirred for 2 h. The reaction was followed by infrared spectroscopy. At the end the solvent was evaporated, the product was dissolved in water and purified by preparative GPC (Sephadex G-25, water as eluent). The final copolymer was isolated by lyophilization.

### Example 8: determination of a lower critical solution temperature (LCST).

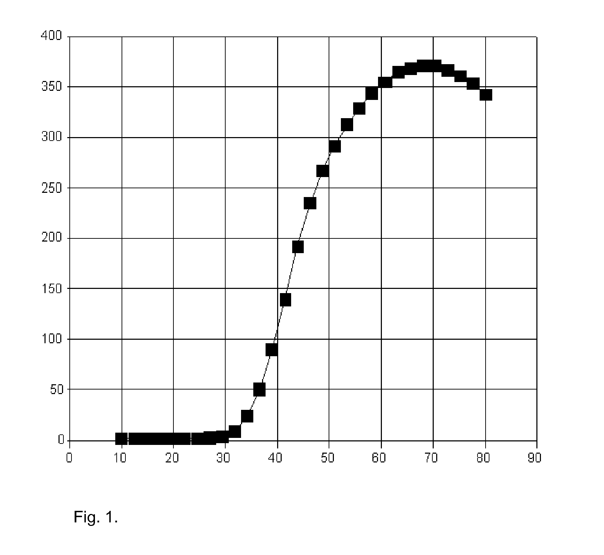

LCST properties (loss modulus G', storage modulus G", and complex viscosity η of the copolymers as a function of temperature) were determined by rheology (oscillation mode) using a Physica MC 301 (Anton Paar) rheometer.
Rheological properties at increasing temperatures were determined using the same polymer concentration as that used in gelling experiments, usually 20 wt%. Figure 1 is a plot of the viscosity (y-axis, in Pa.s) versus temperature (x-axis, in °C). Although rheological measurements actually determined the onset of gelation shown as an increase of viscosity as a function of temperature, we defined the LCST as the temperature at which the viscosity started to increase. In the example of Figure 1, the LCST was 29°C.

### Example 9: in vitro degradation test of thermogels.

The degradation experiments were carried out in 10 mm diameter glass tubes with volume markings. The copolymer was dissolved at 20°C in 10 mM phosphate buffered saline (PBS) at pH 7.4, and at a 20 wt% concentration, or in a 10 mM citric buffer at pH 5.5. 3.0 mL of solution were poured into each tube to ensure a solid gelation.
The glass tubes were placed in an incubator with a shaking bath at 37°C or in a water-bath thermostated at 37°C for 1 h to make the 3 mL solutions gel. Then, 7.0 ml of 10 mM PBS at pH 7.4 or 7.0 ml of a citric buffer to pH 5.5 incubated at the same temperature were placed over the gels. At predetermined time periods, the buffer over the gel was withdrawn and the remaining volume of gel was measured through the volume marking. Then 7.0 mL of fresh buffer pre-incubated at the same temperature were added and the tubes were placed again into the thermostated bath. The remaining gel volumes were plotted against incubation time to get the degradation profiles.

### Example 10: preparation of Paclitaxel-loaded micelles.

Some PHEG-POE-PHEG copolymer and Paclitaxel (1:0.4 w/w) were dissolved in acetonitrile and thoroughly mixed. The solvent was evaporated using a stream of nitrogen under stirring. The mixture was re-dissolved in distilled water and a solution with strong opalescence was obtained. After filtration (G3 filter), the solution was lyophilized. Micelles containing Paclitaxel could be smoothly re-dissolved in water and characterized by light-scattering measurements.

### Example 11: in vitro release of Bovine Serum Albumine (BSA) from a thermogel followed by UV-visible light spectroscopy.

The release experiments were carried out in 10 mm diameter glass tubes. The copolymer was dissolved at 20°C in 10 mM phosphate buffered saline (PBS) at pH 7.4, and at a 20 wt% concentration, or in a 10 mM citric buffer at pH 5.5. BSA at a loading of 1 wt% and 5 wt% was dissolved in the same buffer and mixed with the copolymer solution.
The glass tubes were placed in an incubator with a shaking bath at 37°C or in a water-bath thermostated at 37°C for 1 hour. The dimensions of the gel were 20 mm x 10 mm. Then, 2 ml of 10 mM PBS at pH 7.4 or 2 ml of a citric buffer to pH 5.5 incubated at the same temperature were placed over the gels. At predetermined time periods, the buffer over the gel was withdrawn and replaced with a fresh buffer pre-incubated at the same temperature. The withdrawn samples were analyzed by UV-visible light spectroscopy using the absorption at 494 nm for pH 7.4 and the absorption at 458 nm for pH 5.5.

### Example 12: use of thermogels as temporary void filler and shock absorber in a maxillo-facial trauma.

Upon arrival of a patient to the emergency ward, and after diagnosis of a significant maxillo-facial trauma, a biodegradable thermogel would be injected in the damage areas in order to relieve pain (via an analgesic contained in the composition) and act as a shock absorber between broken bone and tissue parts upon gelation. The gel would also prevent unwanted adhesion of damaged tissue and bones to prevent scar tissue formation. This would give the surgeons more time to plan reconstructive surgery and would cause less trauma for the patient during reconstructive surgery because spontaneous healing would delayed for a few days. By the time the surgeons would be ready, the gel would have started degrading or remaining gel blocks could be removed by cooling them down using cold fluids or instruments and then by sucking the liquefied gel out.

### Example 13: creation of a nerve guide with an inner lining containing growth factors and a lumen containing nerve stem cells.

A tube with an internal diameter matching the external diameter of the nerve to repair was dip-coated on the inside with a cold composition containing an amphiphilic copolymer from this invention (giving a LCST of 15°C), nutrients for cell growth and growth factors. After draining off the excess of composition material, the tube temperature was raised to 20°C to gel the composition as an inner lining against the tube wall. Then the tube lumen was filled by dip-coating with a solution containing nerve stem cells and an amphiphilic copolymer from this invention (giving a LCST of 25°C). The tube temperature was raised to 30°C to gel the solution in the lumen. This tube was implanted at the severed nerve location. Nerve regrowth could occur through rapid erosion of the lumen gel to expose nerve stem cells to the extremities of the severed nerve, and the growth would be sustained by the outer gel coating supplying nutrients and growth factors to the lumen at an optimal rate.

### Example 14: injection of a thermogel containing osteogenic and / or bone morphogenic proteins into intervertebral discs or articulate cartilage to stop or reverse degeneration of diseased or damaged tissues.

A composition of the thermogel with a LCST of 37°C containing amongst other components the growth factor TGF-beta-3, or another osteogenic or bone morphogenic protein was prepared. The composition in its liquid form was injected into the intervertebral disc using a small bore needle or a small diameter cannula. Upon reaching LCST, the composition would gel and hold the growth factor in situ over a period of time, releasing it in a slower manner than straight injection of a non-gelling solution.

## Claims

1. Amphiphilic copolymer, containing at least a hydrophilic chain segment (A) and a hydrophobic chain segment (B), both segments being biodegradable.

2. Amphiphilic copolymer according to claim 1, **characterized in that** the copolymer is a block copolymer or a graft copolymer.

3. Amphiphilic copolymer according to claim 2, **characterized in that** the copolymer is an A(BA)ₓ, B(AB)ₓ or (AB)ₓ block copolymer, block A being the hydrophilic chain segment, block B being the hydrophobic chain segment and x being an integer between 1 and 5.

4. Amphiphilic copolymer according to any one of claims 1 to 3, **characterized in that** the hydrophilic chain segment(s) contains glutamine or aspartamine groups.

5. Amphiphilic copolymer according to any of the claims 1 to 4, **characterized in that** the hydrophilic chain segment(s) contains monomeric units of *N*-(2-hydroxyalkyl)-*L*-glutamine, *L-*glutamic acid, *N*-(2-hydroxyalkyl)-*L*-aspartamine, *L*-aspartic acid, glycolic acid or a combination thereof.

6. Amphiphilic copolymer according to claim 5, **characterized in that** the hydrophilic chain segment(s) is poly[*N*-(2-hydroxyalkyl)-*L*-glutamine], poly[*L*-glutamic acid], poly[*N*-(2-hydroxyalkyl)-*L*-aspartamine], poly[*L*-aspartic acid] or polyglycolide.

7. Amphiphilic copolymer according to any one of claims 1 to 6, **characterized in that** the hydrophobic chain segment(s) contains acetal groups, ortho ester groups or a combination thereof.

8. Amphiphilic copolymer according to any one of claims 1 to 7, **characterized in that** the hydrophobic chain segment(s) contains poly-*L*-lactide, poly-*D*,*L*-lactide, poly(lactide-*co*-glycolide), polyanhydride, polyphosphazene, polyketals.

9. Amphiphilic copolymers according to claims 6 and 7, **characterized in that** copolymer is one out of the group poly[N-(2-hydroxyalkyl)-*L*-glutamine]-polyacetal, poly[N-(2-hydroxyalkyl)-*L*-glutamine]-polyacetal-poly[N-(2-hydroxyalkyl)-*L-*glutamine], polyacetal-poly[N-(2-hydroxyalkyl)-*L*-glutamine]-polyacetal block copolymers, polyacetal-poly[N-(2-hydroxyalkyl)-*L*-glutamine] graft copolymers, poly[N-(2-hydroxyalkyl)-*L*-glutamine]-poly(ortho ester), poly[N-(2-hydroxyalkyl)-*L-*glutamine]-poly(ortho ester)-poly[N-(2-hydroxyalkyl)-*L*-glutamine], poly(ortho ester)-poly[N-(2-hydroxyalkyl)-*L*-glutamine]-poly(ortho ester) block copolymers, poly(ortho ester)-poly[N-(2-hydroxyalkyl)-*L*-glutamine] graft copolymers, poly[N-(2-hydroxyalkyl)-*L*-aspartamine]-polyacetal, poly[N-(2-hydroxyalkyl)-*L*-aspartamine]-polyacetal-poly[N-(2-hydroxyalkyl)-*L*-aspartamine], polyacetal-poly[N-(2-hydroxyalkyl)-*L*-aspartamine]-polyacetal block copolymers, polyacetal-poly[N-(2-hydroxyalkyl)-*L*-aspartamine] graft copolymers, poly[N-(2-hydroxyalkyl)-*L-*aspartamine]-poly(ortho ester), poly[N-(2-hydroxyalkyl)-*L*-aspartamine]-poly(ortho ester)-poly[N-(2-hydroxyalkyl)-*L*-aspartamine], poly(ortho ester)-poly[N-(2-hydroxyalkyl)-*L*-aspartamine]-poly(ortho ester) block copolymers, poly(ortho ester)-poly[N-(2-hydroxyalkyl)-*L*-aspartamine] graft copolymers, poly(L-glutamic acid)-polyacetal, poly(L-glutamic acid)-polyacetal-poly(L-glutamic acid), polyacetal-poly(L-glutamic acid)-polyacetal block copolymers, polyacetal-poly(L-glutamic acid) graft copolymers, poly(L-glutamic acid)-poly(ortho ester), poly(L-glutamic acid)-poly(ortho ester)-poly(L-glutamic acid), poly(ortho ester)-poly(L-glutamic acid)-poly(ortho ester) block copolymers, poly(ortho ester)-poly(L-glutamic acid) graft copolymers, poly(L-aspartic acid)-polyacetal, poly(L-aspartic acid)-polyacetal-poly(L-aspartic acid), polyacetal-poly(L-aspartic acid)-polyacetal block copolymers, polyacetal-poly(L-aspartic acid) graft copolymers, poly(L-aspartic acid)-poly(ortho ester), poly(L-aspartic acid)-poly(ortho ester)-poly(L-aspartic acid), poly(ortho ester)-poly(L-aspartic acid)-poly(ortho ester) block copolymers, poly(ortho ester)-poly(L-aspartic acid) graft copolymers, polyglycolide-polyacetal, polyglycolide-polyacetal-polyglycolide, polyacetal-polyglycolide-polyacetal block copolymers, polyacetal-polyglycolide graft copolymers, polyglycolide-poly(ortho ester), polyglycolide-poly(ortho ester)-polyglycolide, poly(ortho ester)-polyglycolide-poly(ortho ester) block copolymers, poly(ortho ester)-polyglycolide graft copolymers, and combinations thereof.

10. Amphiphilic copolymers according to any one of claims 1 to 9 containing moieties that allow chemical reactions to occur between the polymer chains to achieve polymer crosslinking.

11. Amphiphilic copolymers according to any one of claims 1 to 10 and with a structure compliant to any one of formulas I to VIII.

12. A composition containing at least one amphiphilic copolymer of any one of claims 1 to 11 and at least one therapeutically active agent.

13. A composition according to claim 12, **characterized in that** the copolymer forms micelles in water, the therapeutically active agent being entrapped in the micelles.

14. A composition according to claim 12, **characterized in that** the composition when put in water can form a thermogel with a LCST below 37°C.

15. An implant containing the copolymer according to any one of claims 1 to 11.

16. An implant according to claim 15, the implant containing at least one therapeutically active agent.

17. Compositions including copolymers from any one of claims 1 to 11 and able to form thermogels in water with a LCST below 37°C for use in tissue engineering applications.

18. Compositions including copolymers from any one of claims 1 to 11 and able to form thermogels in water with a LCST below 37°C for use as temporary in vivo void fillers.

19. Use of the compositions of claim 17 or 18 for as temporary void fillers for aesthetic surgery, reconstruction surgery and dental surgery.

20. Use of the compositions of any claim from 15-18 for applications where a swelling of less than 5% in volume is of benefit, such as intra-ocular or intra-cranial surgery.

21. Method of treatment of a human or animal, wherein the composition according to any one of claims 12-20 is administered to the body, preferably by injection.

22. Compositions according to any one of the claims 12-20 where the therapeutically agent is a growth factor.

23. Composition according to claim 22, wherein the transforming growth factor is at least one of the group existing of transforming growth factor beta-3, osteogenic protein 1, bone morphogenic proteins 2 and 7.
